# FASCICULE DE BREVET EUROPEEN

(11) **EP 1 422 236 B1**
(45) Date de publication et mention de la délivrance du brevet: **14.02.2007**
(21) Numéro de dépôt: 03292865.7
(22) Date de dépôt: 19.11.2003
(51) Int. Cl.: C07K 5/06

(54) **Procédé de synthèse du perindopril et de ses sels pharmaceutiquement acceptables**
Verfahren für die Synthese von Perindopril und seiner pharmazeutisch annehmbaren Salzen
Method for synthesis of perindopril and its pharmaceutically acceptable salts

(43) Date de publication de la demande: 26.05.2004
(73) Titulaire: Les Laboratoires Servier, 92415 Courbevoie Cedex (FR)
(72) Inventeur: Dubuffet, Thierry, 76190 Autretot (FR); Langlois, Pascal, 76210 Saint Jean de la Neuville (FR)

(56) Documents cités:
- EP-A- 0 308 341
- SEBLE WAGAW ET AL.: "Palladium-Catalyzed Coupling of Optically active Amines with Aryl Bromides" JOURNAL OF THE AMERICAN CHEMICAL SOCIETY, AMERICAN CHEMICAL SOCIETY, WASHINGTON, DC, US, vol. 119, no. 36, pages 8451-8458, XP002274577 ISSN: 0002-7863

## Description

La présente invention concerne un procédé de synthèse du perindopril de formule (I) : 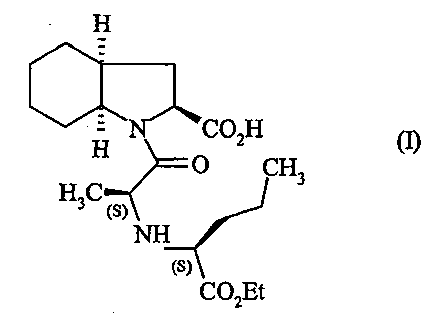 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.

La demanderesse a présentement mis au point un nouveau procédé de synthèse du perindopril à partir de matières premières aisément accessibles.

Plus spécifiquement, la présente invention concerne un procédé de synthèse du perindopril, et de ses sels pharmaceutiquement acceptables, caractérisé en ce que l'on fait réagir le composé de formule (II), de configuration (S) : 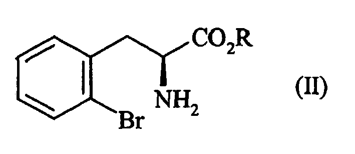 dans laquelle R représente un atome d'hydrogène ou un groupement protecteur de la fonction acide,
soit avec le composé de formule (III) : 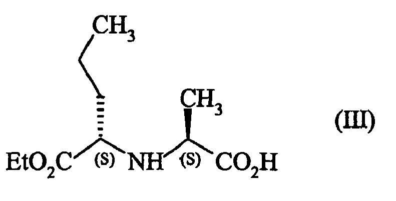 dans des conditions de couplage peptidique,
soit avec le composé de formule (IV) : 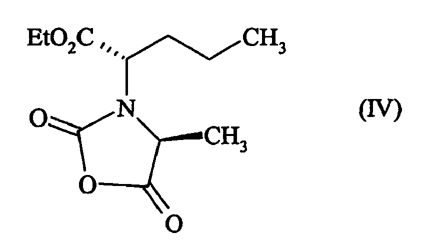 pour conduire au composé de formule (V) : 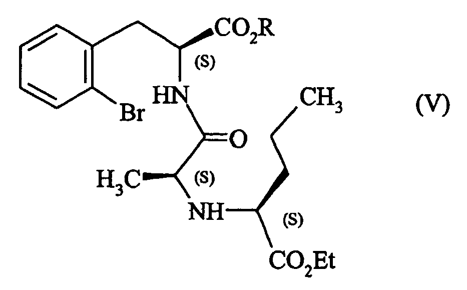 dans laquelle R est tel que défini précédemment,
que l'on soumet à une réaction de couplage intramoléculaire, pour conduire au composé de formule (VI) : 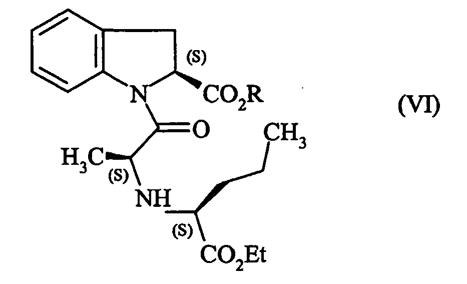 dans laquelle R est tel que défini précédemment,
que l'on soumet à une réaction d'hydrogénation catalytique, pour conduire, après déprotection le cas échéant, au composé de formule (I).

Parmi les groupements protecteurs de la fonction acide, on peut citer à titre non limitatif les groupements benzyle et alkyle (C₁-C₆) linéaire ou ramifié.

La réaction de couplage intramoléculaire est préférentiellement effectuée, soit en présence d'une base et d'un catalyseur à base de palladium, soit à l'aide d'hydrure de sodium et d'iodure de cuivre (I) ou de bromure de cuivre (I).
Les catalyseurs à base de palladium préférentiellement utilisés pour cette réaction de couplage sont les catalyseurs à base de palladium et d'une arylphosphine ou d'une bis-phosphine.

Parmi ces catalyseurs, on peut citer à titre non limitatif Pd(0)/PPh₃, Pd(0)/P(*o*-tolyl)₃, Pd(0)/P(1-naphtyl)₃, Pd(0)/P(*o*-méthoxyphényl)₃, Pd₂(dba)₃/PPh₃, Pd₂(dba)₃/P(*o*-tolyl)₃, Pd₂(dba)₃/P(1-naphtyl)₃, Pd₂(dba)₃/P(*o*-méthoxyphényl)₃, Pd₂(dba)₃/P(2-furyl)₃, Pd₂(dba)₃/dppp, Pd₂(dba)₃/(±)-BINAP et (DPPF)PdCl₂.CH₂Cl₂/DPPF,
étant entendu que par BINAP, on entend 2,2'-bis(diphénylphosphino)-1,1'-binaphtyle, par dba, on entend dibenzylidèneacétone,
par DPPF, on entend 1,1'-bis(diphénylphosphino)ferrocène,
et par dppp, on entend 1,3-bis(diphénylphosphino)propane.

Parmi les bases utilisables pour la réaction de couplage en présence d'un catalyseur à base de palladium, on peut citer à titre non limitatif Cs₂CO₃, NaOtBu, Na₂CO₃, NaOAc et KOAc.

Les composés de formule (V) sont des produits nouveaux, utiles comme intermédiaires de synthèse dans l'industrie chimique ou pharmaceutique, notamment dans la synthèse du perindopril, et font à ce titre partie intégrante de la présente invention.

Les composés de formule (II) peuvent être préparés selon le procédé décrit dans la publication *J*. *Am. Chem. Soc*. **1994,** *116*, 10847-10848.

Le composé de formule (IV) peut être préparé par réaction du composé de formule (III) avec un composé de formule (VII) : 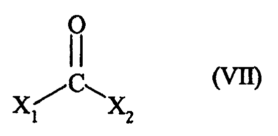 dans laquelle X₁ et X₂, identiques ou différents, représentent chacun un groupement partant tel que, par exemple, un atome d'halogène ou un groupement tosylate, mésylate, alkoxy (C₁-C₆) linéaire ou ramifié, alkylthio (C₁-C₆) linéaire ou ramifié, imidazolyle, benzimidazolyle, tétrazolyle, benzotétrazolyle, trihaloalkyle (C₁-C₆) linéaire ou ramifié, trihaloalkoxy (C₁-C₆) linéaire ou ramifié ou succinimidyloxy.

### EXEMPLE 1 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-2-[(4S)-4-Méthyl-2,5-dioxo-1,3-oxazolidin-3-yl]-pentanoate d'éthyle

Dans un réacteur, charger à 0°C 20 g de N-[(S)-carbéthoxy-1-butyl]-(S)-alanine, 150 ml de toluène, puis 18,4 g de 1,1'-carbonyl-diimidazole, puis amener la température du milieu réactionnel à 20°C. Après 1 heure d'agitation à 20°C, refroidir à nouveau le mélange à 0°C, filtrer le précipité obtenu, puis évaporer le filtrat, pour conduire au produit du titre avec un rendement de 90 %.

### Stade B : Acide (2S)-3-(2-bromophényl)-2-[((2S)-2-{[(1S)-1-(éthoxycarbonyl)butyl]amino}propanoyl)amino]propanoïque

Dans un réacteur, placer 29 g de (*S*)-2-bromophénylalanine et 150 ml de dichlorométhane, puis 18 ml de triéthylamine. Ajouter ensuite lentement une solution de 29 g du composé obtenu au stade précédent dans 50 ml de dichlorométhane, puis agiter 1 heure supplémentaire à température ambiante.
Après addition d'eau, le mélange réactionnel est ensuite refroidi à 15°C et le pH est amené à 4,2 par addition d'une solution d'acide chlorhydrique 2N. Après extraction, les phases organiques sont lavées puis évaporées pour conduire au produit du titre.

### Stade C : Acide (2S)-1-((2S)-2-{[(1S)-1-(éthoxycarbonyl)butyl]amino}propanoyl)-2-indolinecarboxylique :

Dans un réacteur, charger 14,6 g du composé obtenu au stade précédent en solution dans le toluène, 1,57 g de Pd₂(dba)₃, 1,83 g de P(o-tolyl)₃ et 21,5 g de Cs₂CO₃. Amener ensuite le mélange réactionnel à 100°C. Après 15h d'agitation à cette température, le mélange est ramené à température ambiante et purifié par chromatographie sur silice, pour conduire au produit du titre.

### Stade D : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 20 g du composé obtenu dans le stade précédent, en solution dans l'acide acétique, puis 0,5 g de Pd/C à 10 %. Hydrogéner sous pression de 0,5 bars entre 15 et 30°C, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant, pour conduire au produit du titre avec une pureté énantiomérique de 99 %.

### Stade E : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le précipité obtenu dans le stade précédent (20 g) est mis en solution dans 280 ml d'acétate d'éthyle, puis 4 g de tert-butylamine et 40 ml d'acétate d'éthyle sont ajoutés. La suspension obtenue est ensuite portée au reflux jusqu'à dissolution totale, puis la solution obtenue est filtrée à chaud et refroidie sous agitation jusqu'à une température de 15-20°C. Le précipité obtenu est alors filtré, réempâté à l'acétate d'éthyle, séché puis broyé pour conduire au produit du titre avec un rendement de 95%.

### EXEMPLE 2 : (2S)-2-[((1S)-2-{[(1S)-2-(benzyloxy)-1-(2-bromobenzyl)-2-oxoéthyl]amino}-1-méthyl-2-oxoéthyl)amino]pentanoate d'éthyle

Dans un réacteur sont introduits 20 g de (*S*)-2-bromophénylalaninate de benzyle, 240 ml d'acétate d'éthyle puis 8,4 ml de triéthylamine, 8 g de 1-hydroxybenzotriazole, 13 g de N-[(S)-carbéthoxy-1-butyl]-(S)-alanine et 12,3 g de dicylohexylcarbodiimide. Le mélange est ensuite agité à température ambiante pendant 3 heures, puis il est filtré.
Le filtrat est ensuite lavé, puis séché et évaporé à sec pour conduire au produit du titre.

## Revendications

1. Procédé de synthèse du perindopril de formule (I) : 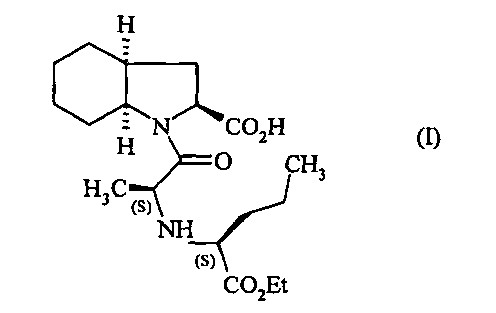 et de ses sels pharmaceutiquement acceptables,
**caractérisé en ce que** l'on fait réagir le composé de formule (II), de configuration (S) : 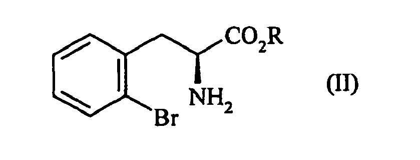 dans laquelle R représente un atome d'hydrogène ou un groupement protecteur de la fonction acide,
soit avec le composé de formule (III) : 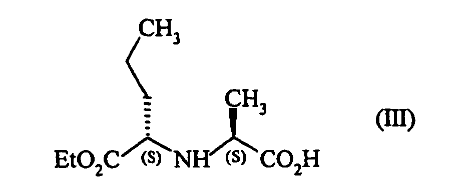 dans des conditions de couplage peptidique, soit avec le composé de formule (IV) : 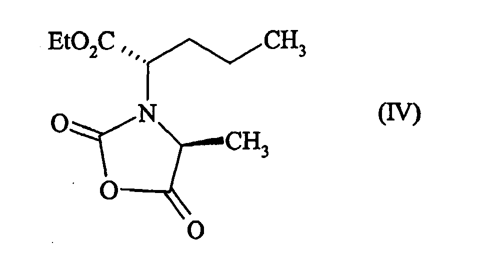 pour conduire au composé de formule (V) : 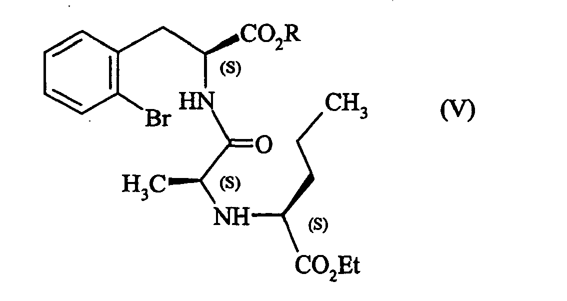 dans laquelle R est tel que défini précédemment,
que l'on soumet à une réaction de couplage intramoléculaire, pour conduire au composé de formule (VI) : 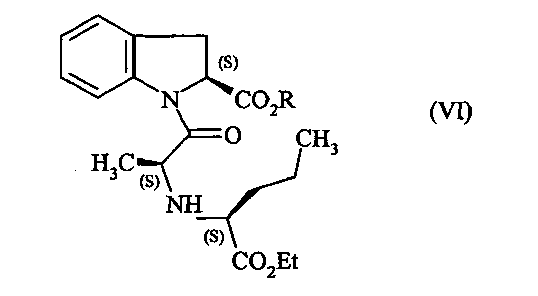 dans laquelle R est tel que défini précédemment,
que l'on soumet à une réaction d'hydrogénation catalytique, pour conduire, après déprotection le cas échéant, au composé de formule (I).

2. Procédé de synthèse selon la revendication 1, **caractérisé en ce que** R représente un groupement benzyle ou alkyle (C₁-C₆) linéaire ou ramifié.

3. Procédé de synthèse selon la revendication 1, **caractérisé en ce que** la réaction de couplage intramoléculaire est effectuée, soit en présence d'une base et d'un catalyseur à base de palladium, soit à l'aide d'hydrure de sodium et d'iodure de cuivre (I) ou de bromure de cuivre (I).

4. Procédé de synthèse selon la revendication 3, **caractérisé en ce que** la réaction de couplage intramoléculaire est effectuée en présence d'une base et d'un catalyseur à base de palladium et d'une arylphosphine ou d'une bis-phosphine.

5. Procédé de synthèse selon la revendication 4, **caractérisé en ce que** la base utilisée pour la réaction de couplage intramoléculaire est choisie parmi Cs₂CO₃, NaOtBu, Na₂CO₃, NaOAc et KOAc.

6. Procédé de synthèse selon la revendication 4 ou 5, **caractérisé en ce que** le catalyseur à base de palladium et d'une arylphosphine ou d'une bis-phosphine est choisi parmi Pd(0)/PPh₃, Pd(0)/P(*o*-tolyl)₃, Pd(0)/P(1-naphtyl)₃, Pd(0)/P(*o*-méthoxyphényl)₃, Pd₂(dba)₃/PPh₃, Pd₂(dba)₃/P(*o*-tolyl)₃, Pd₂(dba)₃/P(1-naphtyl)₃, Pd₂(dba)₃/P(*o-*méthoxyphényl)₃, Pd₂(dba)₃/P(2-furyl)₃, Pd₂(dba)₃/dppp, Pd₂(dba)₃/(±)-BINAP et (DPPF)PdCl₂·CH₂Cl₂/DPPF,
étant entendu que par BINAP, on entend 2,2'-bis(diphénylphosphino)-1,1'-binaphtyle, par dba, on entend dibenzylidèneacétone, par DPPF, on entend 1,1'-bis(diphénylphosphino)ferrocène, et par dppp, on entend 1,3-bis(diphénylphosphino)propane.

7. Composé de formule (V) : 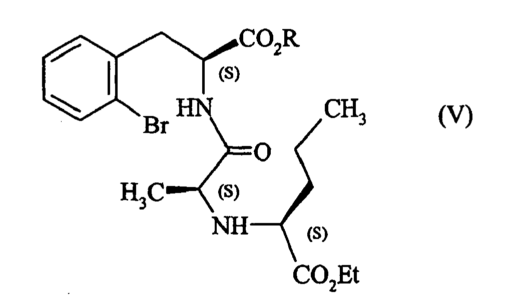 dans laquelle R est tel que défini dans la revendication 1.

8. Procédé de synthèse selon l'une quelconque des revendications 1 à 6 du perindopril sous sa forme de sel de tert-butylamine.

## Claims

1. Process for the synthesis of perindopril of formula (I) : 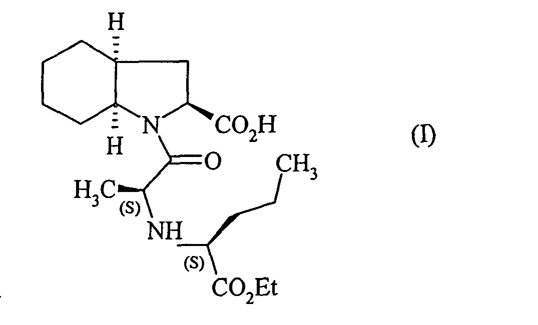 and pharmaceutically acceptable salts thereof,
**characterised in that** the compound of formula (II), of configuration (S) : 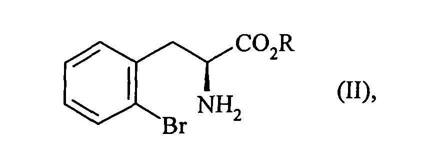 wherein R represents a hydrogen atom or a protecting group for the acid function, is reacted
either with the compound of formula (III): 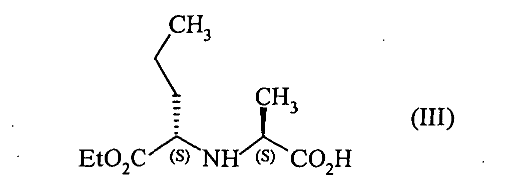 under peptide coupling conditions
or with the compound of formula (IV) : 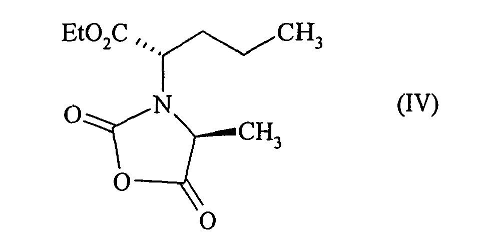 to yield the compound of formula (V) : 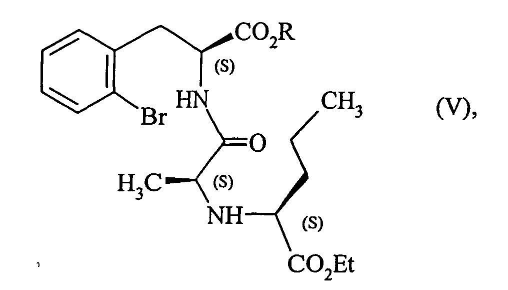 wherein R is as defined hereinbefore,
which is subjected to an intramolecular coupling reaction to yield the compound of formula (VI) : 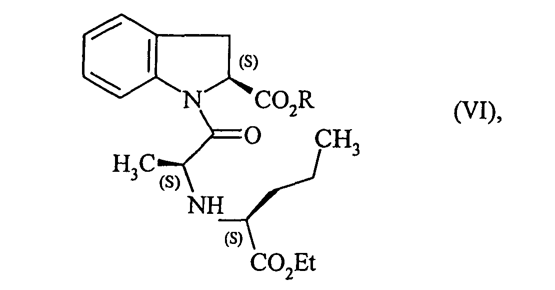 wherein R is as defined hereinbefore,
which is subjected to a catalytic hydrogenation reaction to yield, after deprotection where appropriate, the compound of formula (I).

2. Synthesis process according to claim 1, **characterised in that** R represents a benzyl or linear or branched (C₁-C₆)alkyl group.

3. Synthesis process according to claim 1, **characterised in that** the intramolecular coupling reaction is carried out either in the presence of a base and a catalyst based on palladium or using sodium hydride and copper(I) iodide or copper(I) bromide.

4. Synthesis process according to claim 3, **characterised in that** the intramolecular coupling reaction is carried out in the presence of a base and a catalyst based on palladium and on an arylphosphine or a bisphosphine.

5. Synthesis process according to claim 4, **characterised in that** the base used for the intramolecular coupling reaction is selected from Cs₂CO₃, NaOtBu, Na₂CO₃, NaOAc and KOAc.

6. Synthesis process according to claim 4 or 5, **characterised in that** the catalyst based on palladium and on an arylphosphine or a bisphosphine is selected from Pd(0)/PPh₃, Pd(0)/P(*o*-tolyl)₃, Pd(0)/P(1-naphthyl)₃, Pd(0)/P(*o*-methoxyphenyl)₃, Pd₂(dba)₃/PPh₃, Pd₂(dba)₃/P(*o*-tolyl)₃, Pd₂(dba)₃/P(1-naphthyl)₃, Pd₂(dba)₃/P(*o*-methoxyphenyl)₃, Pd₂(dba)₃/P(2-furyl)₃, Pd₂(dba)₃/dppp, Pd₂(dba)₃/(±)-BINAP and (DPPF)PdCl₂.CH₂Cl₂/DPPF, BINAP being understood to be 2,2'-bis(diphenylphosphino)-1,1'-binaphthyl, dba being understood to be dibenzylideneacetone, DPPF being understood to be 1,1'-bis(diphenylphosphino)ferrocene and dppp being understood to be 1,3-bis(diphenylphosphino)propane.

7. Compound of formula (V) : 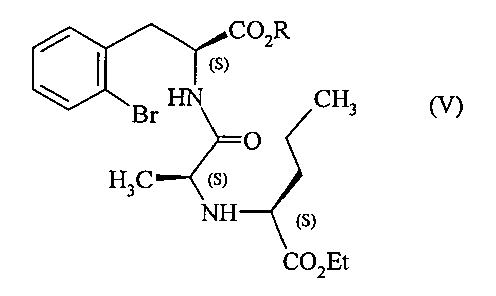 wherein R is as defined in claim 1.

8. Process according to any one of claims 1 to 6 for the synthesis of perindopril in the form of its tert-butylamine salt.

## Patentansprüche

1. Verfahren zur Synthese von Perindopril der Formel (I): 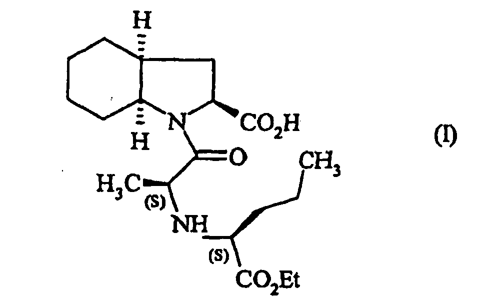 und von seinen pharmazeutisch annehmbaren Salzen,
**dadurch gekennzeichnet, daß** man die Verbindung der Formel (II) in der Konfiguration (S): 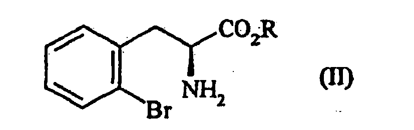 in der R ein Wasserstoffatom oder eine Schutzgruppe für die Säurefunktion bedeutet,
entweder mit der Verbindung der Formel (III): 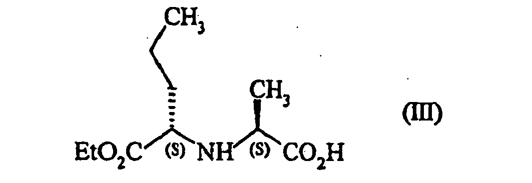 bei Peptidkupplungsbedingungen
oder mit der Verbindung der Formel (IV): 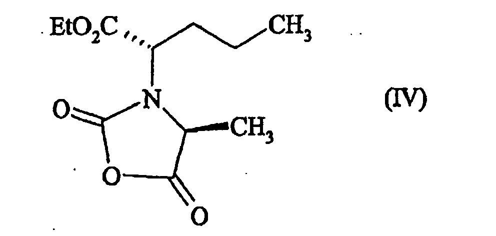 umsetzt zur Bildung der Verbindung der Formel (V): 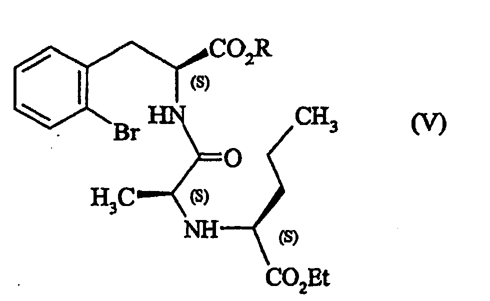 in der R die oben angegebenen Bedeutungen besitzt,
welche man einer intramolekularen Kupplungsreaktion unterwirft zur Bildung der Verbindung der Formel (VI): 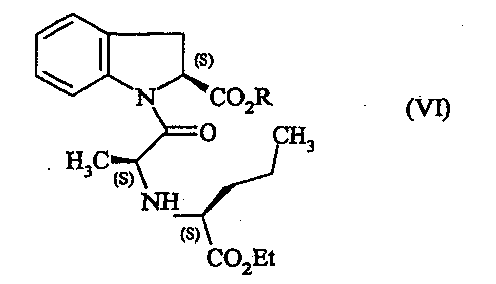 in der R die oben angegebenen Bedeutungen besitzt,
welche man einer katalytischen Hydrierungsreaktion unterwirft, so daß man gegebenenfalls nach der Abspaltung der Schutzgruppe die Verbindung der Formel (I) erhält.

2. Syntheseverfahren nach Anspruch 1, **dadurch gekennzeichnet, daß** R eine Benzylgruppe oder eine geradkettige oder verzweigte (C₁-C₆)-Alkylgruppe bedeutet.

3. Syntheseverfahren nach Anspruch 1, **dadurch gekennzeichnet, daß** die intramolekulare Kupplungsreaktion entweder in Gegenwart einer Base und eines Katalysators auf der Grundlage von Palladium oder mit Natriumhydrid und Kupfer(I)-iodid oder Kupfer(I)-bromid durchgeführt wird.

4. Syntheseverfahren nach Anspruch 3, **dadurch gekennzeichnet, daß** die intramolekulare Kupplungsreaktion in Gegenwart einer Base und eines Katalysators auf der Grundlage von Palladium und eines Arylphosphins oder eines Bisphosphins durchgeführt wird.

5. Syntheseverfahren nach Anspruch 4, **dadurch gekennzeichnet, daß** die für die intramolekulare Kupplungsreaktion verwendete Base aus Cs₂CO₃, NaOtBu, Na₂CO₃, NaOAc und KOAc ausgewählt wird.

6. Syntheseverfahren nach Anspruch 4 oder 5, **dadurch gekennzeichnet, daß** der Katalysator auf der Grundlage von Palladium und eines Arylphosphins oder Bisphosphins aus Pd(O)/PPh₃, Pd(0)/P(*o*-Tolyl)₃, Pd(0)/P(1-Naphthyl)₃, Pd(O)/P(*o*-Methoxyphenyl)₃, Pd₂(dba)₃/PPh₃, Pd₂(dba)₃/P(*o*-Tolyl)₃, Pd₂(dba)₃/P(1-Naphthyl)₃, Pd₂(dba)₃/P(o-Methoxyphenyl)₃, Pd₂(dba)₃/P(2-Furyl)₃, Pd₂ (dba)₃/dppp, Pd₂(dba)₃/(±)-BINAP und (DPPF)PdCl₂.CH₂Cl₂ /DPPF ausgewählt wird, mit der Maßgabe, daß man unter BINAP 2,2'-Bis(diphenylphosphino)-1,1'-binaphthyl, unter dba Dibenzylidenaceton, unter DPPF 1, 1'-Bis(diphenylphosphino)-ferrocen und unter dppp 1,3-Bis(diphenylphosphino)-propan versteht.

7. Verbindung der Formel (V): 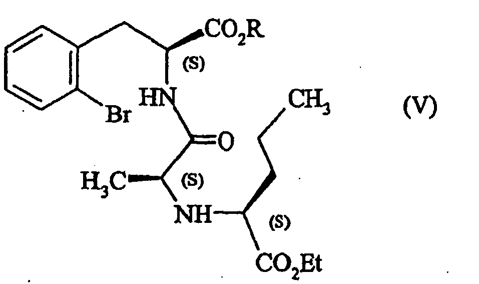 in der R die in Anspruch 1 angegebenen Bedeutungen besitzt.

8. Syntheseverfahren nach einem der Ansprüche 1 bis 6 für die Herstellung von Perindopril in Form seines tert.-Butylaminsalzes.
